# EUROPEAN PATENT APPLICATION

(11) **EP 3 520 706 A1**
(43) Date of publication of application: **07.08.2019**
(21) Application number: 18154477.6
(22) Date of filing: 31.01.2018
(51) Int. Cl.: A61B 8/12, A61B 8/00, A61B 8/06, A61B 8/08, A61N 1/362

(54) **IMPLANT DEVICE FOR IN-BODY ULTRASOUND SENSING**

(71) Applicant: Koninklijke Philips N.V., 5656 AE Eindhoven (NL)
(72) Inventor: HAKKENS, Franciscus Johannes Gerardus, 5656 AE Eindhoven (NL); VAN DER HORST, Arjen, 5656 AE Eindhoven (NL); HENDRIKS, Cornelis Petrus, 5656 AE Eindhoven (NL); KOLEN, Alexander Franciscus, 5656 AE Eindhoven (NL); HILGERS, Achim, 5656 AE Eindhoven (NL); VAN DEN ENDE, Daan Anton, 5656 AE Eindhoven (NL); JOHNSON, Mark Thomas, 5656 AE Eindhoven (NL)
(74) Representative: de Haan, Poul Erik

(57) **Abstract**

An implantable sensing device comprises an electroactive polymer actuator and an ultrasound probe moved by the electroactive polymer actuator. Actuation of the electroactive polymer actuator determines a direction in which the ultrasound probe faces. In this way, the actuator is able to implement a scanning function so that a lower complexity probe (e.g. with fewer transducer elements) may be employed. This saves power and space. The electroactive polymer actuator enables a low power scanning function to be provided.

## Description

### FIELD OF THE INVENTION

This invention relates to implant devices for in-body ultrasound sensing.

### BACKGROUND OF THE INVENTION

There is increasing demand for unobtrusive in-body health sensing and treatment systems. In particular, there is a shift from conventional hospital treatment towards unobtrusive vital signs sensor and actuator technologies, centered around the individual, to provide better information about the subject's general health and to provide personalized treatment functions.

Vital signs monitor systems for example help to reduce treatment costs by disease prevention and enhance the quality of life. They may provide improved physiological data for physicians to analyze when attempting to diagnose a subject's general health condition. Vital signs monitoring typically includes monitoring one or more of the following physical parameters: heart rate, blood pressure, respiratory rate and core body temperature.

It is known to provide implantable sensor devices for monitoring physiological parameters, such as blood pressure. While the initial insertion is an invasive procedure, once this is completed, the sensor remains in place for unobtrusive sensing for a prolonged period of time. Thus, implanted sensor technologies are also seen as minimally invasive (in the long term). It is also known to provide implantable actuator devices, the most obvious example being pacemakers. Implantable blood pressure sensors, restenosis sensors, or actuators for controlled drug delivery based on e.g. a micro-peristaltic pumps have also been proposed.

Implantable devices enable unobtrusive and long term monitoring and treatment of patients with chronic diseases such as heart failure, peripheral artery disease or hypertension.

This invention relates more particularly to sensing using an implant device with an ultrasound probe.

It is known to provide ultrasound Doppler transducers and pulse echo shape determination transducers, and these are known for application on-body as well as in-body by mounting on a catheter or guidewire. Matrix, line or single element ultrasound transducers are available.

There is a need for accurate, local and (semi) continuous monitoring in areas deeper in the body than can be reached by on-body generated ultrasound. Imaging using a catheter or guidewire is an interventional procedure, and an implanted device would have obvious benefits.

However, the implantation of a sensing device imposes additional requirements for low power operation and small size. Scanning of ultrasound beams is normally done electronically, but this requires a matrix array which makes the transducer relatively large and power consuming. For catheters and guidewires there is the option to (manually) rotate or steer the device in order to obtain the correct direction or scan. However, for implantable devices this is not possible.

Thus, there is a problem with known on-body or wearable ultrasound devices that they cannot provide very local information from deeper inside the body. Known in-body ultrasound devices on catheters and guidewires do not provide continuous information over longer time periods. There is also a problem that traditional scanning methods which require relatively large (matrix) transducers that consume a significant amount of power are not suitable for implantation.

There is therefore a need for an implant solution which addresses these problems.

### SUMMARY OF THE INVENTION

The invention is defined by the claims.

According to examples in accordance with an aspect of the invention, there is provided an implantable sensing device, comprising:
an electroactive polymer actuator; and
an ultrasound probe moved by the electroactive polymer actuator, wherein the actuation of the electroactive polymer actuator determines a direction in which the ultrasound probe faces.

This device is able to implement a scanning function so that a lower complexity probe (e.g. with fewer transducer elements) may be employed. This saves power and space. The electroactive polymer actuator enables a low power scanning function to be provided. The direction may be adapted for a best signal, or else ultrasound data from different directions may be combined to create an image with a larger field of view.

The device is implantable in the sense that it can be inserted into the body and left in place for prolonged periods, after the device for insertion (e.g. catheter) has been removed. It is preferably a wireless device, by which is meant there are no wired connections to the implant which are required from outside the body.

The device thus preferably further comprises means for wirelessly transmitting the ultrasound probe sensor data to an external controller. This means may be a coil for inductive coupling with the external controller for inductive coupling, but it could be any wireless communications system, including RF communication (e.g. an RF tag or RF transmitter).

The ultrasound probe for example comprises an array of at most 9 ultrasound transducer elements. Thus, there may be a small array (e.g. 3 x 3) or a small linear array, or else the ultrasound probe may comprise a single ultrasound transducer element.

The ultrasound probe is for example adapted for measurement of Doppler shift and/or pulse echo delay. Thus, the ultrasound function may be used for measuring blood flow or for generating an image from within a vessel.

In a first design, the device comprises a wire fixation for mounting the imaging device within a vessel spaced from the vessel wall, and wherein the electroactive polymer actuator is adapted to extend along a vessel direction, wherein actuation of the electroactive polymer actuator causes bending of a tip, at which the probe is carried, away from the vessel direction.

This design mounts the probe near or at the middle of a vessel so that a central flow may be monitored and/or a surrounding image may be obtained. The probe may face forwards in a relaxed state of the actuator, and the actuator may then bend to direct the tip, at which the probe is mounted, in a different direction. The actuator may be bendable to both sides of the reference direction.

In a second design, there is a fixation for mounting the sensing device adjacent a vessel wall. The actuator may lie flat parallel to the vessel wall in one state, and a free end may then bend away from the vessel wall when actuated. This only needs actuation in one direction from the reference configuration.

The fixation may comprises a stent. The imaging device may then be used for monitoring conditions after a stent procedure, and the device is implanted as part of the stent insertion.

In a third design, the device comprises a connection lead having a chamber for receiving the electroactive polymer actuator and the ultrasound probe. The connection lead is for example a pacemaker lead. The device is then used for monitoring conditions after a pacemaker procedure.

The invention also provides a sensing system, comprising:
an implantable sensing device as defined above; and
a controller for controlling actuation of the electroactive polymer actuator and for receiving the ultrasound data.

This controller may have components which are part of the implanted device and components which are part of an external device which communicates with the implanted parts. Power for the implanted device may be provided by wireless transfer from an external device or there may be a local implanted power supply for the device.

The controller is for example adapted to determine the facing direction of the probe associated with the captured ultrasound data. In this way, the captured ultrasound data can be interpreted based on a known probe facing direction.

The facing direction may simply be derived from knowledge of the actuation signals applied to the electroactive polymer actuator. However, the system may instead comprise a position or shape sensing arrangement associated with the electroactive polymer actuator for providing information concerning the facing direction of the probe.

The controller may be adapted to implement a feedback control system for controlling actuation in dependence on the ultrasound data. By way of example, the actuation may be set to achieve a best image or a flow measurement with the best signal to noise ratio.

### BRIEF DESCRIPTION OF THE DRAWINGS

Examples of the invention will now be described in detail with reference to the accompanying drawings, in which:
Figure 1 shows a first an example of a sensing device for performing Doppler blood flow sensing;
Figure 2 shows a design in which the device is mounted on the wall of a grafted stent for performing pulse echo measurements;
Figure 3 shows a first pair of possible examples of orientations for the ultrasound probe and for deformation of the actuator;
Figure 4 shows a second pair of possible examples of orientations for the ultrasound probe and for deformation of the actuator;
Figure 5 shows a third pair of possible examples of orientations for the ultrasound probe and for deformation of the actuator; and
Figure 6 shows a sensing system comprising the implantable sensing device and a controller.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an implantable sensing device, comprising an electroactive polymer actuator and an ultrasound probe moved by the electroactive polymer actuator. Actuation of the electroactive polymer actuator determines a direction in which the ultrasound probe faces. In this way, the actuator is able to implement a scanning function so that a lower complexity probe (e.g. with fewer transducer elements) may be employed. This saves power and space. The electroactive polymer actuator enables a low power scanning function to be provided.

The device may be for measuring flow or for measuring distances (and hence providing an imaging function) or both.

For flow measurement, the device is preferably mounted near the middle of the vessel, whereas for imaging, the device may be located at one side wall and measure distances across the vessel to an opposite side wall.

The device is implantable, i.e. it can be left in the body to perform its sensing function and then sends the sensor data to an external controller.

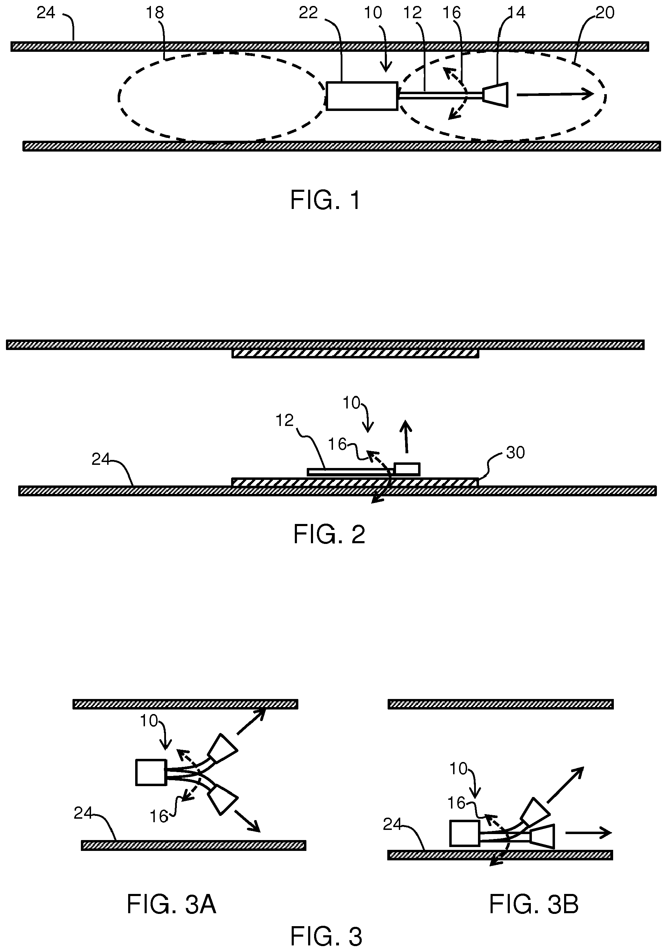

Figure 1 shows a first example of the sensing device applied as an implanted device for performing Doppler blood flow sensing.

The implantable sensing device 10 comprises an electroactive polymer actuator 12 and an ultrasound probe 14 carried by the electroactive polymer actuator 12. The actuation of the electroactive polymer actuator causes bending as represented by arrow 16 and this determines a direction in which the ultrasound probe faces.

The ultrasound probe may comprise a single ultrasound transducer element, or it may comprise a small array, for example with at most 9 ultrasound transducer elements. This array may be a linear array. In this example, the ultrasound probe is adapted for measurement of Doppler shifts for measurement of a flow velocity.

A wire fixation is provided, comprising first and second wire loops 18, 20 which retain a main support part 22 at a fixed location and orientation within the vessel, in particular spaced from the vessel wall 24, such as an artery wall. The wire loops are for example formed of a super-elastic shape memory alloy, so that they may be compressed for insertion into the vessel by catheterization and then released to adopt their retaining shape.

The device 10 is mounted with the electroactive polymer actuator 12 extending along the vessel direction. In a rest position, the ultrasound probe 14 faces the vessel direction. Actuation of the electroactive polymer actuator causes bending at its tip, at which the probe 14 is carried, away from the vessel direction.

In this way, the ultrasound probe may be aligned in the optimal direction for the blood flow measurement, for example a direction which gives rise to the highest signal to noise ratio. The electroactive polymer actuator may then be retained in that optimal bending state. In this way, the actuator is operated with feedback control, the ultrasound signal functioning as an input to the feedback control loop.

The Doppler flow sensing may be combined with pulse echo shape (or shape change) measurement for determining the artery diameter. For the purposes of interpreting the received images, the direction in which the probe faces is determined from the known shape of the actuator, either known from the applied actuation signal (e.g. voltage or current) or additionally measured by sensing, for example using a sensing layer (such as polyvinylidene fluoride, PVDF) in the layer stack of the electroactive polymer actuator. A hybrid configuration may for example be based on an integrated ionic polymer sensor. In this way the direction of the generated ultrasound signal is known.

The device may additionally perform pressure sensing, either using the same transducer elements as for ultrasound measurements, or using an additional dedicated pressure sensor. Even when separate ultrasound transducers and pressure sensors are used, they may still be of the same type, for example a capacitive micromachined ultrasound transducer (CMUT) may have two CMUT elements, one for ultrasound and the other for pressure sensing.

The single transducer element, or multiple transducer elements, of the device may be based on lead zirconate titanate (PZT), a single crystal transducer element, PVDF, or CMUT cells. An ultrasound generating polymer layer may even be integrated into the electroactive polymer actuator multilayer stack.

No matter how many transducer elements are used, ultrasound pulses are generated and received. The Doppler shift is detected to determine the blood flow velocity whereas the pulse echo time delay is measured to determine the distance between the transducer element and tissue interfaces where there are acoustic impedance steps.

The electroactive polymer actuator may be able to deform in one plane (so that the direction of the probe is scanned within one plane) or in multiple planes (so that the direction of the ultrasound probe can be scanned in three dimensions).

3D scanning is for example of interest to map the shape of organs, such as the inside of the heart. Doppler flow may be measured in different directions. 3D scanning may be implemented by using at least 2 actuators.

The electroactive polymer actuators have the advantage of being small and thin and have low power consumption, which is crucial in an implantable application.

The generated ultrasound can be shaped in a parallel beam or it can be focused by applying a lens. By combining directional information, from the actuator, with pulse echo delay distance information for tissue interfaces, organs can be mapped. This can be combined with the Doppler frequency shift to enable a 3D plot of blood velocity and organ shape.

The measurements are taken periodically rather than continuously, to save power and to enable long term monitoring, such as over days, weeks or even months or years. A local power source may be provided which uses energy harvesting or else a wireless recharging capability may be provided. Some of the options for the powering of the device and the transmission of signals are discussed below.

Figure 2 shows a design in which the device 10 is mounted on the wall of a grafted stent 30 for example after abdominal aortic aneurysm repair. The size of the aneurysm may be measured by pulse echo measurements.

In this example, the stent functions as a fixation for mounting the imaging device adjacent a vessel wall. The ultrasound probe faces diametrically across the vessel in the relaxed state, to measure distances across the vessel. However the direction is scanned (as shown by arrow 16). The directional information of the actuator is combined with the measured pulse echo time delay thereby determining the shape and size of the aneurism. In this way the aneurysm growth or growth rate may be determined thereby checking the performance of the stent graft (leakage) and risk of rupture.

The shape measurement may again be combined with Doppler flow measurement. The actuator may for example align the transducer probe more parallel to the stent graft when Doppler flow is to be measured. The blood flow measurement may be used to provide an indication of the performance of the stent graft.

There are various options for orientation of the ultrasound probe and for deformation of the actuator.

Figure 3 shows a first pair of possible examples.

Figure 3A corresponds to the configuration of Figure 1. The probe faces a direction parallel with the vessel, which is optimal for flow velocity measurement, and is located near the center of the vessel. Deformation of the actuator redirects the probe to face the vessel wall so that distance measurements may be obtained.

Figure 3B shows the device mounted at the side wall of the vessel. The probe still faces a direction parallel with the vessel, which is suitable for flow velocity measurement, although the flow measurement is less optimal since it is measured near the side wall. Deformation of the actuator redirects the probe to face (at an angle) the opposing vessel wall so that distance measurements may be obtained.

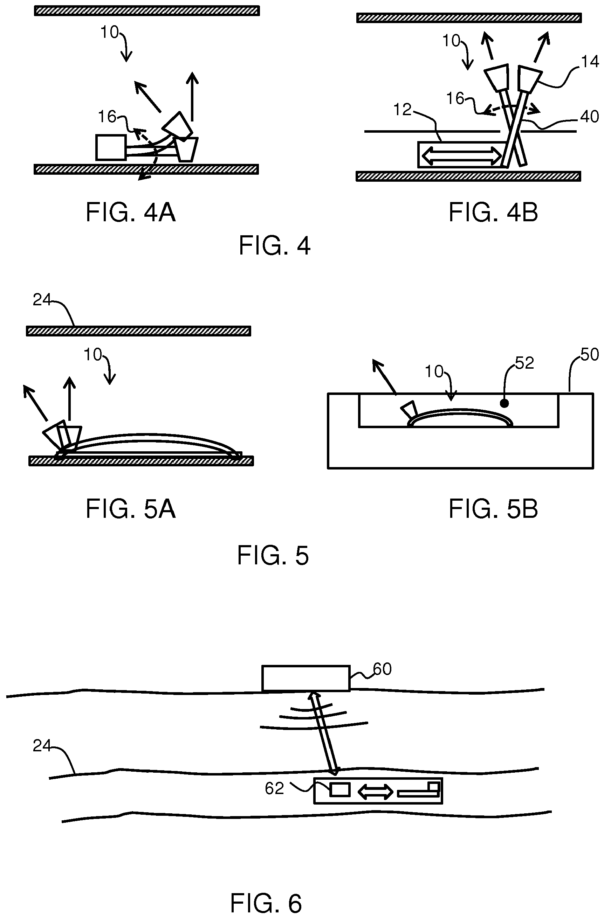

Figure 4 shows a second pair of possible examples.

Figure 4A corresponds to the configuration of Figure 2. The device is mounted at the side wall of the vessel. The probe faces a direction perpendicular to the vessel, i.e. across the diameter, to measure the distance to structures across the vessel width. Deformation of the actuator redirects the probe so that 3D volume information may be measured, and this provides a better direction for any Doppler measurements.

Figure 4B shows a version where the actuator does not directly carry the probe. The actuator 12 implements a linear expansion or contraction instead of performing bending, and it pushes on a support 40 to provide a decoupled actuation. Thus, this example shows that the probe may not be mounted directly on the actuator, but a more complex linkage mechanism may be provided. However, the probe is still moved by the actuator.

Figure 5 shows a third pair of possible examples.

Figure 5A shows another version with device mounted at the side wall of the vessel. The actuator is fixed at both ends, instead of having one free end as in some of the examples above. Actuation causes a bowing and thereby shape change. The probe is mounted on the actuator so that this shape change translates to a change in probe direction. The probe initially faces a direction perpendicular to the vessel, i.e. across the diameter, to measure the distance to structures across the vessel width. Deformation of the actuator redirects the probe so that 3D volume information may be measured, and this provides a better direction for Doppler measurements.

Figure 5B shows a version where the device is integrated in the lead 50 of a pacemaker. This enables long term monitoring of heart volume and blood flow (pump capacity progression). The device 10 is formed in a sealed chamber 52 within the lead 50. Multiple devices in multiple directions can be integrated. Power and data processing can be integrated in the pulse generator of the pacemaker.

The actuation in the examples above is always based on the use of an electroactive polymer actuator. A shape memory alloy actuator may additionally be used, for example for course alignment, for instance immediately after placement, with the later scanning function then implemented by the electroactive polymer actuator. This provides a coarse and fine actuation approach. This functionality may also be used to lock the actuator into a better position when the position for optimal Doppler signal reception (as determined following a scan using the electroactive polymer actuator) is detected.

The device only has to measure during short time periods. During periods of inactivity, the device can scavenge energy. The actuator can be made very flexible when not actuated and hence wobbles in the bloodstream but then stiffens up when in measurement mode. This can for example be achieved by forcing the actuator into a bi-stable mode for the measurement period and releasing for the non-measurement period. The use of bi-stable EAP actuators is described in WO 2016/193412. The advantage is that that only energy is required to switch from one to the other state. Another possible approach is to scan continuously in a resonance wobbling mode partly driven be the heart cycle, and that part of the motion is used to scavenge energy.

Figure 6 shows a sensing system comprising the implantable sensing device 10 as described above and a controller 60, 62 for controlling actuation of the electroactive polymer actuator and for receiving the ultrasound data.

The controller comprises some parts 60 which are outside the body and some parts 62 that are inside the body as part of the implant. The sensor data is transferred between the two controller parts. There are various options for the powering of the device 10 and for the transmission of signals, and some of these are discussed below. For example, the implanted controller parts 62 may be powered by an internal power source and/or by receiving power wirelessly using a receiver. The external controller parts 60 have an external power source 22 and optionally also a wireless power transmitter.

As mentioned above, the device may perform blood flow pressure sensing in addition to the ultrasound sensing. This may be performed using the structure of the electroactive polymer actuator. A sensor-actuator may be used to allow simultaneous sensing and actuation. For an IMPC sensor-actuator, this can be achieved by measuring the impedance of the outer electrodes separately to the actuation voltage or adding a high frequency signal to the quasi-dc actuation signal. The approach of combining a DC actuation signal with a high frequency superposed AC signal for sensing is described in detail in WO 2017/036695.

This AC ripple is provided by the control circuit 62 and the same circuit monitors the electrical properties of the EAP element which are a measure for the blood flow pressure.

The circuit 62 may in one example receive electrical energy via an electrical coil from a remote power source which is part of the circuit 60, and via the same coil it can send the measured pressure, distance and/or flow values. Thus, use may be made of only an external power source.

However it may be chosen to integrate a battery as well into the implantable device; this will depend on the capacity of a miniature battery and the required functional time of the devices. Instead of, or in addition to, using a battery as an implanted power source, an energy harvesting device can be used which extracts energy from the human body and transforms this energy into electricity. As such, the devices can be operated for prolonged time.

As mentioned above, there are various options for wireless connection to the implanted sensor, either to provide a remote source of power, or to provide a communication channel or both.

In general an implant, whether passive or active, may be powered in many ways. Depending on the functionality and operation mode of the implant, different requirements for the energy source are present.

For a continuous active function, such as a requirement for active mechanical actuation in order to generate an output signal, there is a higher energy requirement than for a passive temporally limited (e.g. on-demand) function, such as the occasional read out of an active sensor or occasional change to a stable actuator. However in both cases, there is a need for either a wired connection to a local power source, or a wireless coupling to a power transmitter.

Delivering electrical power to medical implants for powering or communication is a topic which is well-described in literature.

Comprehensive reviews of power aspects for implantable medical devices are given in B. A. Achraf, A. B. Kouki and C. Hung, "Power Approaches for Implantable Medical Devices," sensors, no. 28889-28914; doi:10.3390/s151128889, 2015, J. Lee, J. Jang and Y.-K. Song, "A review on wireless powering schemes for implantable microsystems in neural engineering applications," Biomed Eng Letters, no. DOI 10.1007/s13534-016-0242-2, pp. 6:205-215, 2016, A. Kim, M. Ochoa, R. Rahim and B. Ziaie, "New and Emerging Energy Sources for Implantable Wireless Microdevices," IEEE: SPECIAL SECTION ON NANOBIOSENSORS, no. 10.1109/ACCESS.2015.2406292, 2014, and K. N. Bocan and E. Sejdi'c, "Adaptive Transcutaneous Power Transfer to Implantable Devices: A State of the Art Review," sensors, vol. 16, no. doi:10.3390/s16030393, p. 393, 2016.

Any of these solutions may be used to power the implant, and some approaches will be discussed below.

A first approach is to provide a wired power source as part of the implant. A wired power source may be an ordinary battery (non-rechargeable or rechargeable), directly connected to the implant or to its operating electronics. However, since implants usually will be worn over a long period of time, a high capacity and high energy density battery would be of benefit. The power density of (re-chargeable) batteries is expected to grow further making them increasingly suitable for long term monitoring functions.

Instead of conventional batteries, bio-fuel cells or nuclear batteries may be applicable. Another alternative power source, which is very similar to a battery, is a super capacitor, which is a capacitor having an extremely high capacitance and a very low self-discharge characteristic.

Energy harvesters may instead be used to operate any implant. Accordingly a power generator could for example be operated by human body energy such as motion of an extremity but also motion of an inner organ or any dynamics resulting from a fluid flow (blood in an artery) or gas. The power generator may be able to store energy in a super capacitor or re-chargeable battery, and/or be able to directly operate an implant.

An energy harvester does not necessarily need to be in close vicinity to the implant itself but could also be spatially separated. A wired connection may be used between them. Also in the field of energy harvesters, efforts are being made to make them smaller and more efficient in order to make them more attractive as an internal (and everlasting) energy source for medical devices.

Wireless energy transmission systems may be classified according to the physical coupling mechanism, which can be either capacitive, inductive (magnetic) or electromagnetic. All three mechanisms have their own pros and cons and preferred applications. In general, the performance of each approach depends very much on specific boundary conditions such as e.g. the size of the transmitter- and receiver-element (which can be a plate, an inductor or an antenna) and the distance and medium between both elements, as well as their orientation with respect to each other.

An additional smart feature of all wireless power systems is the intrinsic ability of a bidirectional data communication between a transmitter and a receiver.

In applications where low energy levels at short distances need to be transmitted, capacitive coupling may be used. Low to medium power levels at medium to long range may be preferably realized via an electromagnetic coupling. Highest power levels at short distances may be transmitted via an inductive coupling, making use of magnetic fields.

A most basic approach only enables the ultrasound data to be gathered when the external controller is present. However, using such a wireless powering technique would not necessarily imply the need to wear such a transmitter continuously to perform the intended use of the implant. For example, an implant may only need to be operated during certain treatments (in e.g. a hospital) or it may only need to be activated at predefined moments in time (e.g. morning, afternoon, evening).

An alternative use case would be to use such a wireless transmitter overnight, to charge an implanted power source, which would be used to operate an implant during the day. This is a hybrid approach where there is a local energy supply so sensor data can be gathered and stored in memory without an external controller in place, but it has a short duration so needs recharging periodically.

The implanted wireless receiver unit and the implanted sensor and/or actuator may be spatially separated from each other. For example, the receiving element, e.g. a receiver inductance may be located directly underneath the skin, in order to realize a strong coupling between the transmitter and receiver and thus to maximize the energy transmission efficiency and to minimize the charging time of an implanted battery. Of course, this would require a more involved implantation procedure than if the implanted elements are fully integrated into the stent (or other support structure).

There are also options which do not rely on electrical energy to realize a wireless energy transmission system, in particular making use of optical, ultrasonic or mechanical pressure waves.

As discussed above, a controller performs the data processing and control. The controller can be implemented in numerous ways, with software and/or hardware, to perform the various functions required. A processor is one example of a controller which employs one or more microprocessors that may be programmed using software (e.g., microcode) to perform the required functions. A controller may however be implemented with or without employing a processor, and also may be implemented as a combination of dedicated hardware to perform some functions and a processor (e.g., one or more programmed microprocessors and associated circuitry) to perform other functions.

Examples of controller components that may be employed in various embodiments of the present disclosure include, but are not limited to, conventional microprocessors, application specific integrated circuits (ASICs), and field-programmable gate arrays (FPGAs).

In various implementations, a processor or controller may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor or controller.

As explained above, the actuator (optionally with a sensing function as well) is implemented using an electroactive polymer (EAP) device. EAPs are an emerging class of materials within the field of electrically responsive materials. EAPs can work as sensors or actuators and can easily be manufactured into various shapes allowing easy integration into a large variety of systems.

Materials have been developed with characteristics such as actuation stress and strain which have improved significantly over the last ten years. Technology risks have been reduced to acceptable levels for product development so that EAPs are commercially and technically becoming of increasing interest. Advantages of EAPs include low power, small form factor, flexibility, noiseless operation, accuracy, the possibility of high resolution, fast response times, and cyclic actuation.

The improved performance and particular advantages of EAP material give rise to applicability to new applications. An EAP device can be used in any application in which a small amount of movement of a component or feature is desired, based on electric actuation or for sensing small movements.

The use of EAPs enables functions which were not possible before, or offers a big advantage over common sensor / actuator solutions, due to the combination of a relatively large deformation and force in a small volume or thin form factor, compared to common actuators. EAPs also give noiseless operation, accurate electronic control, fast response, and a large range of possible actuation frequencies, such as 0 - 1MHz, most typically below 20 kHz.

Devices using electroactive polymers can be subdivided into field-driven and ionic-driven materials.

Examples of field-driven EAPs include Piezoelectric polymers, Electrostrictive polymers (such as PVDF based relaxor polymers) and Dielectric Elastomers. Other examples include Electrostrictive Graft polymers, Electrostrictive paper, Electrets, Electroviscoelastic Elastomers and Liquid Crystal Elastomers.

Examples of ionic-driven EAPs are conjugated/conducting polymers, Ionic Polymer Metal Composites (IPMC) and carbon nanotubes (CNTs). Other examples include ionic polymer gels.

Field-driven EAPs are actuated by an electric field through direct electromechanical coupling. They usually require high fields (tens of megavolts per meter) but low currents. Polymer layers are usually thin to keep the driving voltage as low as possible.

Ionic EAPs are activated by an electrically induced transport of ions and/or solvent. They usually require low voltages but high currents. They require a liquid/gel electrolyte medium (although some material systems can also operate using solid electrolytes).

Both classes of EAP have multiple family members, each having their own advantages and disadvantages.

A first notable subclass of field driven EAPs are Piezoelectric and Electrostrictive polymers. While the electromechanical performance of traditional piezoelectric polymers is limited, a breakthrough in improving this performance has led to PVDF relaxor polymers, which show spontaneous electric polarization (field driven alignment). These materials can be pre-strained for improved performance in the strained direction (pre-strain leads to better molecular alignment). Normally, metal electrodes are used since strains usually are in the moderate regime (1-5%). Other types of electrodes (such as conducting polymers, carbon black based oils, gels or elastomers, etc.) can also be used. The electrodes can be continuous, or segmented.

Another subclass of interest of field driven EAPs is that of Dielectric Elastomers. A thin film of this material may be sandwiched between compliant electrodes, forming a parallel plate capacitor. In the case of dielectric elastomers, the Maxwell stress induced by the applied electric field results in a stress on the film, causing it to contract in thickness and expand in area. Strain performance is typically enlarged by pre-straining the elastomer (requiring a frame to hold the pre-strain). Strains can be considerable (10-300%). This also constrains the type of electrodes that can be used: for low and moderate strains, metal electrodes and conducting polymer electrodes can be considered, for the high-strain regime, carbon black based oils, gels or elastomers are typically used. The electrodes can again be continuous, or segmented.

A first notable subclass of ionic EAPs is Ionic Polymer Metal Composites (IPMCs). IPMCs consist of a solvent swollen ion-exchange polymer membrane laminated between two thin metal or carbon based electrodes and requires the use of an electrolyte. Typical electrode materials are Pt, Gd, CNTs, CPs, Pd. Typical electrolytes are Li+ and Na+ water-based solutions. When a field is applied, cations typically travel to the cathode side together with water. This leads to reorganization of hydrophilic clusters and to polymer expansion. Strain in the cathode area leads to stress in rest of the polymer matrix resulting in bending towards the anode. Reversing the applied voltage inverts bending. Well known polymer membranes are Nafion® and Flemion®.

Another notable subclass of Ionic polymers is conjugated/conducting polymers. A conjugated polymer actuator typically consists of an electrolyte sandwiched by two layers of the conjugated polymer. The electrolyte is used to change oxidation state. When a potential is applied to the polymer through the electrolyte, electrons are added to or removed from the polymer, driving oxidation and reduction. Reduction results in contraction, oxidation in expansion.

In some cases, thin film electrodes are added when the polymer itself lacks sufficient conductivity (dimension-wise). The electrolyte can be a liquid, a gel or a solid material (i.e. complex of high molecular weight polymers and metal salts). Most common conjugated polymers are polypyrrole (PPy), Polyaniline (PANi) and polythiophene (PTh).

An actuator may also be formed of carbon nanotubes (CNTs), suspended in an electrolyte. The electrolyte forms a double layer with the nanotubes, allowing injection of charges. This double-layer charge injection is considered as the primary mechanism in CNT actuators. The CNT acts as an electrode capacitor with charge injected into the CNT, which is then balanced by an electrical double-layer formed by movement of electrolytes to the CNT surface. Changing the charge on the carbon atoms results in changes of C-C bond length. As a result, expansion and contraction of single CNT can be observed.

For the sensing functionality, the use of capacitance change is one option, in particular in connection with an ionic polymer device. For field driven systems, a capacitance change can also be measured directly or by measuring changes in electrode resistance as a function of strain.

Piezoelectric and electrostrictive polymer sensors can generate an electric charge in response to applied mechanical stress (given that the amount of crystallinity is high enough to generate a detectable charge). Conjugated polymers can make use of the piezo-ionic effect (mechanical stress leads to exertion of ions). CNTs experience a change of charge on the CNT surface when exposed to stress, which can be measured. It has also been shown that the resistance of CNTs change when in contact with gaseous molecules (e.g. O₂, NO₂), making CNTs usable as gas detectors.

Sensing may also be based on force measurements and strain detection. Dielectric elastomers, for example, can be easily stretched by an external force. By putting a low voltage on the sensor, the strain can be measured as a function of voltage (the voltage is a function of the area).

The device of the invention has various possible uses. In particular, there are various situations where there is a clinical need for in-body measurements which can be performed by the device described above. The device is able to measure blood flow velocity in arteries or veins (using Doppler-based, hemodynamic sensing), inside stents, inside the heart (in the pump capacity) or in aneurysms after placing a stent graft. The device may also be used to determine the shape of in-body lumens, organs, etc. by means of pulse echo measurements. For example, the size of an aneurysm can be monitored over a long time scale, thereby detecting growth. The heart chamber volume can be measured which relates to pump capacity.

The amount of fluid around the heart can be measured, thereby monitoring progression of CHF (congestive heart failure). The size of an artery and the growth rate of plaque can be monitored by measuring pulse echo transit time from blood to plaque and plaque to the vessel wall based on the multiple reflections which result from the acoustic impedance steps. These can be combined together with the actuator shape information in a velocity and shape plot. The device can be a dedicated implantable or be an add-on to an implantable device with another main function, such as a stent, heart valve, pacemaker (on the leads), heart support pump etc. The device can use its own power supply or else it may make use of an existing power supply and communication channel, for example which may already exist such as in the case of combination with a heart support pump.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. An implantable sensing device (10), comprising:
an electroactive polymer actuator (12); and
an ultrasound probe (14) moved by the electroactive polymer actuator, wherein the actuation of the electroactive polymer actuator determines a direction in which the ultrasound probe (14) faces.

2. A device as claimed in claim 1, further comprising means (62) for wirelessly transmitting the ultrasound probe sensor data to an external controller (60).

3. A device as claimed in claim 1 or 2, wherein the ultrasound probe (14) is carried by the electroactive polymer actuator (12).

4. A device as claimed in any preceding claim, wherein the ultrasound probe comprises an array of at most 9 ultrasound transducer elements.

5. A device as claimed in any preceding claim, wherein the ultrasound probe comprises:
a linear array of ultrasound transducer elements; or
a single ultrasound transducer element.

6. A device as claimed in any preceding claim, wherein the ultrasound probe is adapted for measurement of Doppler shift and/or pulse echo delay.

7. A device as claimed in any preceding claim, comprising a wire fixation (18) for mounting the imaging device within a vessel spaced from the vessel wall, and wherein the electroactive polymer actuator is adapted to extend along a vessel direction, wherein actuation of the electroactive polymer actuator causes bending of a tip, at which the probe is carried, away from the vessel direction.

8. A device as claimed in any one of claims 1 to 6, comprising a fixation (30) for mounting the sensing device adjacent a vessel wall.

9. A device as claimed in claim 8, wherein the fixation (30) comprises a stent.

10. A device as claimed in any one of claims 1 to 6, comprising a connection lead (50) having a chamber for receiving the electroactive polymer actuator and the ultrasound probe.

11. A device as claimed in claim 10, wherein the connection lead (50) is a pacemaker lead.

12. A sensing system, comprising:
an implantable sensing device as claimed in any preceding claim; and
a controller (60) for controlling actuation of the electroactive polymer actuator and for receiving the ultrasound data.

13. A system as claimed in claim 12, wherein the controller is adapted to determine the facing direction of the probe associated with the captured ultrasound data.

14. A system as claimed in claim 13, comprising a position or shape sensing arrangement associated with the electroactive polymer actuator for providing information concerning the facing direction of the probe.

15. A system as claimed in claim 12, 13 or 14, wherein the controller is adapted to implement a feedback control system for controlling actuation in dependence on the ultrasound data.
